(19) Europäisches Patentamt
European Patent Office
Office européen des brevets

(11) Veröffentlichungsnummer: **0 048 476**
**B1**

(12) # EUROPÄISCHE PATENTSCHRIFT

(45) Veröffentlichungstag der Patentschrift: **27.06.84**

(51) Int. Cl.³: **C 07 C 51/31**, C 07 C 55/02, C 07 C 55/14

(21) Anmeldenummer: **81107443.4**

(22) Anmeldetag: **19.09.81**

(54) **Verfahren zur kontinuierlichen Herstellung von gesättigten, aliphatischen Polycarbonsäuren in einem Siedereaktor.**

(30) Priorität: **23.09.80 DE 3035809**

(43) Veröffentlichungstag der Anmeldung: **31.03.82 Patentblatt 82/13**

(45) Bekanntmachung des Hinweises auf die Patenterteilung: **27.06.84 Patentblatt 84/26**

(84) Benannte Vertragsstaaten: **BE CH DE FR GB IT LI LU NL SE**

(56) Entgegenhaltungen:
**DE - A - 1 224 729**
**DE - A - 2 435 387**
**US - A - 2 459 690**

(73) Patentinhaber: **CHEMISCHE WERKE HÜLS AG, Postfach 1320, D-4370 Marl 1 (DE)**

(72) Erfinder: **Heumann, Hans, Dr., Hander Weg 18, D-5100 Aachen (DE)**
Erfinder: **Hilt, Walter, Overkamps-Hof 12, D-4690 Herne 1 (DE)**
Erfinder: **Liebing, Heinz, Königsstrasse 13, D-4690 Herne 2 (DE)**
Erfinder: **Schweppe, Manfred, Overweg 34, D-4690 Herne 1 (DE)**

(74) Vertreter: **Steil, Hanna, Dipl.-Chem. et al, RSP PATENTE - PB 15 Postfach 1320, D-4370 Marl 1 (DE)**

**EP 0 048 476 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Wenn man bei der Herstellung von Dicarbonsäuren durch Oxydation von cycloaliphatischen Alkoholen oder Ketonen mit wäßriger Salpetersäure die Bildung von kürzerkettigen Säuren in Grenzen halten will, ist die Einhaltung eines engen Temperaturbereiches während der Oxydation notwendig. Weitere Erschwernisse ergeben sich durch Verkrustungen auf den Kühlflächen, die durch auskristallisierende organische Säuren hervorgerufen werden. Dadurch muß man bei diskontinuierlicher Fahrweise zum Ende der Reaktion hin die Temperatur erheblich ansteigen lassen, um die Abführung der Reaktionswärme sicherstellen zu können.

Bei kontinuierlichen Verfahren, z. B. in einer Rührkesselkaskade, läßt sich ein wesentlich engerer Temperaturbereich (±2 bis 4° C) realisieren, wenn der Gehalt der organischen Säuren im Oxydationsgemisch, das Verhältnis Salpetersäure : Wasser, ein großer Salpetersäureüberschuß und eine geringe Temperaturdifferenz zwischen Reaktionsgemisch und Kühlwasser eingehalten werden.

Außerdem wurde empfohlen, die Oxydation bei einem derart verminderten Druck durchzuführen, daß das Reaktionswasser unter Ausnützung der Reaktionswärme bei der optimalen Oxydationstemperatur von 60—65° C verdampft. Diese Aufgabe sollte durch den Einsatz einer Verdampferapparatur mit einem Kolonnenaufsatz zur Trennung von Salpetersäure und Wasserdampf gelöst werden. Dabei muß jedoch der Rektifizierkolonne Wärme zugeführt werden, damit das Energiedefizit gedeckt werden kann, außerdem fehlen bei diesem Verfahren Maßnahmen zur Schaumvermeidung.

Überraschenderweise wurde nun gefunden, daß man die Mängel der bisher geschilderten Salpetersäure-Oxydationsverfahren vermeiden und dabei auch noch zu höheren Dicarbonsäureausbeuten und zu einer kontinuierlichen Arbeitsweise gelangen kann, wenn man die Oxydation der jeweiligen Cycloverbindungen

bei Unterdruck in einem Siedereaktor durchführt,
der aus einem Kessel ohne Rührer und ohne Kühlelemente,
aber mit aufgesetzter Rektifizierkolonne und gegebenenfalls einem Brüdenabscheider besteht,
wobei man mit Hilfe von Kondensierapparaten,
korrosionsbeständigen Gebläsen und Trennapparaturen unter Ausnutzung der Oxydationswärme und unter Umpumpen von stickoxydhaltigem Schleppgas,
dem gegebenenfalls Sauerstoff oder sauerstoffhaltige Gasgemische zugesetzt werden,
Rückführkreisläufe mit teilweiser Austragung für die Haupt- und Nebenprodukte bildet,
und schließlich die reinen Oxydationsprodukte isoliert.

Das erfindungsgemäße Verfahren zur kontinuierlichen Herstellung von aliphatischen Polycarbonsäuren ist in den Ansprüchen 1 bis 6 beschrieben.

Die Oxydation der gegebenenfalls alkylsubstituierten Cyclo-Verbindung erfolgt also (vergl. Figur) in einem Kessel K, dem kontinuierlich mit Hilfe von Dosierpumpen und Reglern für das Mengenverhältnis die Cyclo-Verbindung und sogenannte Verfahrenssäure mit einem $HNO_3$-Gehalt von 40 bis 55 Gew.-% zugeführt werden. Das Mengenverhältnis von Cyclo-Verbindung und Salpetersäure wurde so geregelt, daß ein Molverhältnis von 1 : 3 bis 20 eingehalten wurde. Die besten Ergebnisse wurden bei Molverhältnissen zwischen 1 : 6 bis 12 beobachtet.

Die Konzentration der Salpetersäure kann in relativ weiten Grenzen variiert werden. Die günstigsten Ergebnisse bei der Oxydation von Cyclohexanol wurden erzielt, wenn die dem Kessel zugeführte Säure einen $HNO_3$-Gehalt von 40—55 Gew.-% besaß. Bei der Oxydation von Trimethylcyclohexanol empfehlen sich unter Umständen niedrigere Gehalte, beispielsweise von 40—50 Gew.-%.

Die zugeführte Säure bestand in der Regel nur zum Teil aus Frischsäure, vielmehr setzt sich die Verfahrenssäure zusammen aus gegebenenfalls geringer konzentrierter Rückgewinnungssäure von verschiedenen Anfallorten (siehe unten) und entsprechend höher konzentrierter Frischsäure.

Die Reaktionspartner werden entweder durch Wasserdampf oder — nach Ablauf der Startphase — durch Reaktionsabgase, bzw. die anschließend kontinuierlich abgezogenen Anteile der Sumpfphase auf 40 bis 70° C vorgewärmt.

Im Kessel K wird durch geeignete Vorrichtungen, vorzugsweise mit einer Flüssigkeitsringpumpe P ein Unterdruck von 40 bis 200 mbar erzeugt und damit gleichzeitig ein Schleppgas eingesaugt bzw. umgepumpt, das vorzugsweise aus Stickoxyden, Sauerstoff und gegebenenfalls Ballastgasen wie Stickstoff besteht. Der jeweils gewünschte Unterdruck wird durch geeignete Regelvorrichtungen innerhalb enger Grenzen konstant gehalten.

Außerdem können die üblichen Katalysatoren bzw. Katalysatorgemische dem Siedereaktor zugeführt werden. Bei der erfindungsgemäß durchgeführten Oxydation hat sich ein Gemisch von Kupfer und Ammoniumvanadat besonders bewährt. Üblicherweise wurde ein Kontaktspiegel von je 1000 bis 1500 mg Cu bzw. $NH_4VO_3$/kg Säure eingehalten. Dabei wurde der untere Wert niemals unterschritten.

Beim Kontakt der Reaktionspartner bei der Siedetemperatur beim jeweiligen Unterdruck tritt praktisch sofort die quantitative Oxydation der Cyclo-Verbindung ein, z. B. bei Cyclohexanol gemäß der Formel:

$$C_6H_{11}OH + 4\,HNO_3 \rightarrow HOOC - (CH_2)_4 - COOH + 2\,NO + 2\,NO_2 + 3\,H_2O$$

bzw. bei Cyclohexanon gemäß

$$C_6H_{10}O + 4\,HNO_3 \rightarrow HOOC - (CH_2)_4 - COOH + NO + 3\,NO_2 + 2\,H_2O$$

Daneben werden gegebenenfalls ein oder mehrere C-Atome des aufgesprengten Ringes bis zu $CO_2$ oxydiert, so daß als Nebenprodukte bei der Cyclohexanol/Cyclohexanon-Oxydation unter Umständen auch

| | |
|---|---|
| Glutarsäure | $HOOC - (CH_2)_3 - COOH$ oder |
| Bernsteinsäure | $HOOC - (CH_2)_2 - COOH$ im |

Oxydationsgemisch zu finden sind.

In analoger Weise setzen sich auch alkylierte oder in anderer Weise substituierte Cyclohexanole und Cyclohexanone um, z. B. Trimethylcyclohexanol zu Trimethyladipinsäure. Es läßt sich jedoch voraussehen, daß sich auch andere Oxydationsreaktionen im Siedereaktor durchführen lassen werden.

Die Stickoxyde des Schleppgases bilden sich also bei der Reaktion selbst. Sie werden mit Hilfe der korrosionsbeständigen Gebläsepumpe G ständig im Kreislauf geführt, wobei durch Zusatz von Sauerstoff oder sauerstoffhaltigen Gasen vor dem Einleiten in den Sumpf des Kessels und auch am Fuß der Rektifizierkolonne (siehe unten) eine laufende Aufoxydation erfolgt.

Beim Einsaugen von Stickstoffdioxyd in die Sumpfphase des Reaktors bildet sich neben salpetriger Säure laufend Salpetersäure, die ihrerseits wieder die jeweilige Cyclo-Verbindung oxydiert.

Die Verwendung von Stickoxyden zur Oxydation der Cyclo-Verbindungen, bzw. die Rückführung von Stickoxyden in das Oxydationsgemisch mit Salpetersäure gehört allerdings zum Stand der Technik. Neu ist jedoch die Verwendung als Schleppgas im Siedereaktor.

In der auf den Kessel K des Siedereaktors bzw. auf einem entsprechenden Brüdenabscheider B mit den Verbindungen E und A zum Kessel K, aufgesetzten Rektifizierkolonne R werden gegebenenfalls anfangs mitgerissene Anteile der organischen Oxydationsprodukte und der Cyclo-Verbindung niedergeschlagen und entweder direkt oder über das Rücklaufrohr E des Brüdenabscheiders wieder in den Reaktionskessel K zurückgeführt.

Zusätzliche Wärmeaustauschmedien werden in der Rektifizierkolonne R nicht verwendet, jedoch muß ihre Bodenzahl (bzw. im einfachsten Fall ihre Baulänge) so bemessen werden, daß organische Verbindungen sicher zurückgehalten werden.

Außerdem läßt man zweckmäßigerweise Kondensat, das an anderer Stelle des Verfahrens gewonnen wird (siehe unten), den aufsteigenden Dämpfen und Gasen entgegensprühen und/oder -rieseln.

Zur Verbesserung der Oxydation von NO zu $NO_2$ wird gegebenenfalls am unteren Ende der Rektifizierkolonne R Sauerstoff oder ein sauerstoffhaltiges Gasgemisch in die Gasphase eingesaugt.

Stickoxyde, Sauerstoff und die Inertbestandteile des Schleppgases und gegebenenfalls $CO_2$ verlassen gemeinsam mit den Dämpfen von Wasser (und $HNO_3$ und/oder $HNO_2$) das obere Ende der Rektifizierkolonne.

Diese Top-Gase und -Dämpfe werden gegebenenfalls mehrstufigen Kondensierapparaten C zugeleitet, in denen (mindestens in deren erster Stufe $C_1$) die flüssigen (noch isolierten) Ausgangsstoffe, vorzugsweise die Verfahrenssäure, als Austauschflüssigkeit aufgewärmt werden.

In späteren Stufen des Kondensationsapparates $C_2$ werden gegebenenfalls auch Kühlmedien mit möglichst tiefen Austauschtemperaturen eingesetzt, d. h. das jeweils zur Kühlung zur Verfügung stehende Brauchwasser.

In der Vorlage V sammelt sich ein Stickoxyd-(bzw. $HNO_2$- und $HNO_3$-)haltiges Wasser an, das mit Hilfe einer Pumpe dem oberen Ende der Rektifizierkolonne teilweise wieder zugeführt wird, (siehe oben) und dessen Überschüsse gegebenenfalls einer Salpetersäure-Absorptions-Anlage als Aufgabewasser für die Türme bzw. Kolonnen zugeführt werden.

Anstelle der empfohlenen Flüssigkeitsringpumpe zur Erzeugung des Unterdrucks im Siedereaktor-System können gegebenenfalls auch Dampfstrahler oder andere korrosionsbeständige Saugpumpen verwendet werden.

Die ausgeschleuste Gasmischung besteht vorwiegend aus dem Ballastgas, das bei der Sauerstoffzugabe zum Schleppgas übrigbleibt, gewöhnlich also aus Stickstoff sowie aus kleineren Mengen von Sauerstoff, Stickoxyden und Kohlensäure.

Diese Gasmischung führt man zweckmäßigerweise in die Gasphase einer Salpetersäure-Absorptions-Anlage zur weiteren Verwertung ein. Bei geringen Stickoxydgehalten wird man das Restgas — gegebenenfalls nach Zusatz weiteren Verdünnungsgases — unter Umständen auch verwerfen können, insbesondere dann, wenn eine Salpetersäure-Anlage nicht zur Verfügung steht.

Im Kessel K kann man auf die Verwendung eines Rührwerkes verzichten, da der (durch die Arbeitsbedingungen ausgelöste und unterhaltene) Siedevorgang und das »eingeblasene« Schleppgas für eine hinreichende Turbulenz sorgen.

Die Temperatur wird im Oxydationsgemisch im Kessel K, sowie an mehreren Stellen der Rektifizierkolonne gemessen. Selbstverständlich werden die Gase und Flüssigkeiten der Zu- und Abgänge bei

allen Wärmeaustauschern $C_1$, $C_2$ und U (siehe unten) ebenfalls kontrolliert. Schließlich ist auch eine Dampfleitung vorgesehen, über die Dampf direkt in den Kessel K eingeleitet werden kann, damit in der Startphase des Prozesses ein rasches Aufheizen des Reaktionsgemisches auf die optimale Oxydationstemperatur möglich ist. Dieser Zusatzdampf wird jedoch gewöhnlich nur nach Betriebsunterbrechungen benötigt, wenn die Reaktionspartner nicht genügend vorerwärmt zugeführt werden können. Sobald der Wärmeaustausch in den entsprechenden Einrichtungen (siehe oben) in Gang gekommen ist, wird diese Dampfleitung meistens nicht mehr benötigt. Gegebenenfalls lassen sich die Ausgangsstoffe auch durch eine Außenheizung in entsprechender Weise vorwärmen.

Der Flüssigkeitsstand im Kessel K ist durch geeignete Vorrichtungen konstant zu halten, z. B. durch eine Exzenter-Schneckenpumpe.

Im Sumpf des Kessels K sammelt sich eine wäßrige Lösung der Oxydationsprodukte mit Gehalten von Rest-$HNO_3$ an. Sie wird kontinuierlich über das Sperrsystem S mit einer Pumpe Z, z. B. einer Mohnopumpe, abgezogen und erwärmt dabei in einem Wärmeaustauscher U die zugeführten Ausgangsstoffe, insbesondere die jeweils zu oxydierenden Cyclo-Verbindungen.

Die aus dem System ausgeschleuste Flüssigkeit kann mit Hilfe der üblichen Trennverfahren in die Bestandteile zerlegt werden.

Bei Adipinsäure gelingt die erste Trennung am einfachsten chargenweise durch Ablassen der ausgeschleusten Flüssigkeit in einen wassergekühlten Kristaller. Nach genügender Verweilzeit, z. B. von 90 Minuten, erhält man durch Filtration ein Rohkristallisat mit einer Restfeuchte von größenordnungsmäßig 20% und Restsäure.

Bei Trimethyladipinsäure ist — bei ausreichend niedrigem $HNO_3$-Gehalt — eine einfache Trennung der organischen und wäßrigen Phase (mit der Restsäure) möglich.

Die Restsäure setzt man zweckmäßigerweise der Frischsäure und dem aus dem Kondensator der Rektifizierkolonne anfallenden Säure zu, die nach entsprechender Aufwärmung durch die Reaktionsprodukte in den Kessel eingespeist wird.

Durch weitere Trennverfahren lassen sich aus dem Rohkristallisat bzw. aus der organischen Phase schließlich weitere Restsäuren und die einzelnen Oxydationsprodukte isolieren. Bei der Oxydation von Cyclohexanol und/oder Cyclohexanon sind das vor allem Adipinsäure und geringe Mengen von Glutar- und Bernsteinsäure, bei Trimethylcyclohexanol die entsprechenden Trimethyldicarbonsäuren. (Die bei der Raffination anfallende Restsäure wird genauso wieder verwendet, wie die bei der ersten Trennung der organischen Phase angefallene Restsäure.)

## Versuchsbeispiele

In einer Versuchsanlage mit einem Kessel von 6 l Inhalt und einer Rektifizierkolonne von 2 m Länge und 9,0 cm Durchmesser mit einem Brüdenabscheider von 6 l Inhalt und einer Rücklaufleitung von der Nennweite 25 und einer beheizten Absaugleitung von ebenfalls 25 mm Durchmesser wurden 20 kontinuierlich gefahrene Versuche von jeweils 8 Stunden Dauer durchgeführt.

Beim kontinuierlichen Durchsatz von 1,5 l Cyclohexanol/h und 11,5 l Verfahrenssäure/h (von 50—56% $HNO_3$) (Mengenverhältnis 1 kg/11,5 kg) ergab sich im Reaktor eine Verweilzeit von etwa 20 Minuten.

Wurde bei einem dieser Versuche die Cyclohexanolzufuhr unterbrochen, so hörte die Gasabspaltung praktisch momentan auf, und der Umlauf brach zusammen. Gleichzeitig konnte ein sofort einsetzender Temperaturabfall beobachtet werden. Diese Beobachtung beweist, daß die Reaktion selbst sehr rasch verläuft.

In den Reaktorbehälter bzw. unterhalb der Rektifizierkolonne wurden 1—2 $m^3$/h Luft eingeblasen.

Die in der Vorlage V kondensierte wäßrige Phase wurde teilweise dem Kopf der Kolonne als Rücklauf zugeführt und teilweise ausgeschleust. Das Rücklaufverhältnis lag mit 0,7 kg Rücklauf/h, 2 bis 3 kg Kopfabzug/h bei 1 : 3 bis 1 : 4.

Über den Behälter W wurden 0,3 bis 2 $m^3$ Abgas/Stunde aus einer zum Reaktor zurückgeführten Kreisgasmenge von etwa 1 bis 6 $m^3$/h ausgeschleust.

In der in den Kessel eingespeisten Säure reicherten sich die organischen Säuren bis auf etwa 6—7% (infolge des Zusatzes der hinter U anfallenden Restsäure) an. Eine Ausschleusung von Restsäure war jedoch nicht erforderlich, denn die genannte Grenze wurde auch bei langer Versuchsdurchführung nicht überschritten.

Der organische Anteil der Verfahrenssäure hatte etwa folgende Zusammensetzung:

| | |
|---|---|
| Vorläufe | 3,7% |
| Bernsteinsäure | 13,9% |
| Glutarsäure | 46,8% |
| Adipinsäure | 31,6% |
| Nachläufe | 4,0% |

Über die Abzugspumpe wurden unter Konstanthaltung des Flüssigkeitsstandes im Reaktorbehälter ca. 12 l Oxydationsgemisch/Stunde abgezogen. Über eine beheizte Leitung, in der das Material auf

70—80° C aufgeheizt wurde, gelangte das Gemisch in einen Kristaller. Die Kristallisationstemperatur lag bei etwa 22° C und die Abtrennung des Rohkristallisates von der Mutterlauge erfolgte über Nutschen. Stündlich fielen etwa 12,2 kg Restsäure und 2,9 kg Rohkristallisat mit ca. 20% Restfeuchte an.

Das aus dem Reaktorbehälter abgezogene Oxydationsgemisch enthält etwa

19—21% organische Säuren
37—41% Salpetersäure
40—42% Wasser

Der Anteil der organischen Säuren hat dabei folgende Zusammensetzung:

| | |
|---|---|
| Adipinsäure | 77,3% |
| Glutarsäure | 17,1% |
| Bernsteinsäure | 5,6% |

Bei der Bilanzierung der 20 Versuche ergab sich, unter Berücksichtigung der organischen Bestandteile in der Verfahrenssäure, bezogen auf das eingesetzte Hexanol eine Adipinsäure-Ausbeute von ca. 95,5%.
Je kg Adipinsäure wurden etwa 1,1 kg $HNO_3$ verbraucht.

Als Vorteile bei dem neuen Verfahren können folgende Punkte festgehalten werden:

1. Kontinuierliches (nicht chargenweises) Verfahren der Oxydation
2. Einsparung von $HNO_3$
3. volle Ausnutzung der Reaktionsenergie
4. Selbstregelung des Verfahrens durch Niveau-, Druck- und Temperaturregelung möglich
5. hohe Ausbeute an Dicarbonsäure

**Patentansprüche**

1. Verfahren zur kontinuierlichen Herstellung von gesättigten, aliphatischen Polycarbonsäuren durch Oxydation von gegebenenfalls alkylsubstituierten Cycloalkanolen und/oder Cycloalkanonen mit Salpetersäure bei erhöhter Temperatur, gegebenenfalls in Gegenwart von Oxydationskatalysatoren und Aufarbeitung des resultierenden Reaktionsgemisches, dadurch gekennzeichnet, daß man die Oxydation der jeweiligen Cyclo-Verbindungen
bei Unterdruck in einem Siedereaktor durchführt,
der aus einem Kessel ohne Rührer und ohne Kühlelemente,
aber mit aufgesetzter Rektifizierkolonne und gegebenenfalls einem Brüdenabscheider besteht,
wobei man mit Hilfe von Kondensierapparaten,
korrosionsbeständigen Gebläsen und Trennapparaturen unter Ausnutzung der Oxydationswärme
und unter Umpumpen von stickoxydhaltigem Schleppgas,
dem gegebenenfalls Sauerstoff oder sauerstoffhaltige Gasgemische zugesetzt werden,
Rückführkreisläufe mit teilweiser Austragung für die Haupt- und Nebenprodukte bildet
und schließlich die reinen Oxydationsprodukte isoliert.

2. Verfahren nach Anspruch 1, dadurch gekennzeichnet, daß man in den Kessel des Siedereaktors bei 40 bis 200 mbar und bei den entsprechenden Siedetemperaturen des Reaktionsgemisches

a) Verfahrenssäure mit 40 bis 55 Gew.-% $HNO_3$,
b) die zu oxydierende Cyclo-Verbindung, wobei beide Reaktionspartner nach Ablauf der Startphase durch Reaktionsabgase vorgewärmt werden,
c) den Katalysator,
d) das Schleppgas und gegebenenfalls — insbesondere zum Einleiten der Reaktion —
e) Wasserdampf einleitet bzw. einbläst,

und man am Kopf der Rektifizierkolonne Stickoxyde und Restsauerstoff und die Inertgasbestandteile des Schleppgases sowie die Dämpfe absaugt, und man die Dämpfe in Kondensierapparaten, in denen gleichzeitig die zugeführten Reaktionspartner aufgewärmt werden, niederschlägt und teilweise wieder am Kopf der Rektifizierkolonne als Rücklauf einspeist oder als Stickoxyd-haltiges Wasser einer Salpetersäure-Absorptionsanlage zuführt, während man den Hauptteil der gasförmigen Bestandteile mit Hilfe eines korrosionsbeständigen Gebläses und unter Zusatz von Sauerstoff oder eines sauerstoffhaltigen Gasgemisches, gegebenenfalls erneut aufgewärmt, als Schleppgas in den Sumpf des Kessels des Siedereaktors zurückleitet, und man ihren Rest ausschleust und gegebenenfalls einer Salpetersäure-Absorptionsanlage zuführt und man aus dem Sumpf des Kessels oder vom Boden des Brüdenabscheiders kontinuierlich Flüssigkeit, gegebenenfalls unter Aufwärmung, abzieht, aus der man nach den üblichen Trennverfahren Salpetersäure abtrennt, die man der Verfahrenssäure vor dem Aufwärmen im Kondensierapparat zusetzt, und man schließlich nach weiteren Trenn- und Reinigungsverfahren neben geringen Mengen weiterer Salpetersäure, die man ebenfalls als Zusatz zur Verfahrenssäure verwenden kann, die Polycarbonsäuren in reiner Form gewinnt.

3. Verfahren nach den Ansprüchen 1 und 2, dadurch gekennzeichnet, daß man bei den zugeführten Reaktionspartnern ein Molverhältnis von 1 : 3 bis 20, vorzugsweise von 1 : 6 bis 12, zwischen Cyclo-Verbindung und $HNO_3$ einhält.

4. Verfahren nach einem oder mehreren der Ansprüche 1 bis 3, dadurch gekennzeichnet, daß man als in den Siedereaktor einzuführende Verfahrenssäure ein Gemisch verwendet, das aus

a) der bei der Reinigung der zu isolierenden Oxydationsprodukte anfallenden Restsäure und
b) sogenannter Frischsäure besteht,

wobei man die gegebenenfalls niedrigere Konzentration der nach a) anfallenden Säure durch entsprechend höhere Konzentration der Frischsäure b) auf den bei der Verfahrenssäure erwünschten Gehalt von 40 bis 55 Gew.-% $HNO_3$ bringt.

5. Verfahren nach einem oder mehreren der Ansprüche 1 bis 4, dadurch gekennzeichnet, daß man zur Erzeugung des Unterdrucks im Siedereaktor vorzugsweise eine Flüssigkeitsringpumpe mit geschlossenem Flüssigkeitskreislauf verwendet.

6. Verfahren nach einem oder mehreren der Ansprüche 1 bis 5, dadurch gekennzeichnet, daß man zusätzlich Luft oder Sauerstoff am untersten Boden der Rektifizierkolonne oder in den Sumpf des Brüdenabscheiders einsaugt.

## Claims

1. Process for the continuous production of saturated aliphatic polycarboxylic acids by oxidising cycloalkanols and/or cycloalkanones, which may be alkyl-substituted, with nitric acid at an elevated temperature, if appropriate in the presence of oxidation catalysts, and working up the resulting reaction mixture, characterized in that the oxidation of the particular cyclo compounds is carried out under reduced pressure in a boiling reactor which consists of a vessel without a stirrer and without cooling elements but with an attached rectifying column and, if appropriate, a vapour separator, recycle circulations with partial discharge of the main products and by-products being formed with the aid of condensing apparatus, corrosion-resistant blowers and separation apparatus, utilising the heat of oxidation and circulating a carrier gas which contains nitric oxide and to which, if appropriate, oxygen or oxygen-containing gas mixtures are added, and the pure oxidation products being finally isolated.

2. Process according to claim 1, characterized in that

a) process acid with 40 to 55% by weight of $HNO_3$,
b) the cyclo compound to be oxidised, both reaction partners being preheated by the reaction exit gases after the end of the starting phase,
c) the catalyst,
d) the carrier gas and if appropriate — in particular for initiating the reaction,
e) steam are introduced or blown

into the vessel of the boiling reactor at 40 to 200 mbar and at the corresponding boiling temperatures of reaction mixture, nitric oxides and residual oxygen and the inert gas constituents of the carrier gas as well as the vapours are extracted at the top of the rectification column, and the vapours are precipitated in condensing apparatus, where simultaneously the reaction partners fed in are heated up, and partially recycled as reflux to the top of the rectification column or fed as water containing nitric oxide to a nitric acid absorption unit, whilst most of the gaseous constituents are, with the aid of a corrosion-resistant blower and with the addition of oxygen or an oxygen-containing gas mixture, heated up again if appropriate, passed back as carrier gas into the bottom of the vessel of the boiling reactor and the remainder thereof is discharged and, if appropriate, fed to a nitric acid absorption unit and liquid, if appropriate with heating, is continuously taken off from the bottom of the vessel or the bottom of the vapour separator, from which liquid nitric acid is separated off by the conventional separation processes, this nitric acid being added to the process acid before heating-up in the condensing apparatus, and finally the polycarboxylic acids are obtained in a pure form after further separation and purification processes, in addition to small amounts of further nitric acid which can also be used as an addition to the process acid.

3. Process according to claims 1 and 2, characterized in that a molar ratio of 1 : 3 to 20, preferably 1 : 6 to 12, between the cyclo compound and $HNO_3$ is maintained in the reaction partners fed in.

4. Process according to one or more of claims 1 to 3, characterized in that, as the process acid to be introduced into the boiling reactor, a mixture is used which consists of

a) the residual acid obtained in the purification of the oxidation products to be isolated and
b) so-called fresh acid,

the concentration, which may be lower, of the acid obtained according to a) being brought to the desired content of 40 to 55% by weight of $HNO_3$ in the process acid by means of a correspondingly higher concentration of the fresh acid b).

5. Process according to one or more of claims 1 to 4, characterized in that, for generating the reduced pressure in the boiling reactor, a liquid ring pump with a closed liquid circulation is preferably used.

6. Process according to one or more of claims 1 to 5, characterized in that air or oxygen are additionally drawn into the lowest plate of the rectification column or into the bottom of the vapour separator.

**Revendications**

1. Procédé pour la fabrication en continu d'acides polycarboxyliques aliphatiques, saturés, par oxydation de cycloalcanols et/ou de cycloalcanones, éventuellement substitués par un alcoyl, avec de l'acide nitrique à température élevée, éventuellement en présence de catalyseurs d'oxydation et traitement du mélange réactionnel résultant, procédé caractérisé en ce que l'on exécute l'oxydation des composés cycliques voulus sous pression réduite dans un réacteur bouilleur, qui est constitué d'un bouilleur sans agitateur ni éléments refroidissants, mais portant une colonne de rectification et, éventuellement un séparateur de buées, où l'on forme, à l'aide d'appareils condenseurs, de ventilateurs résistants à la corrosion et d'appareils de séparation, en tirant parti de la chaleur d'oxydation et en remettant en circulation, au moyen d'une pompe, le gaz porteur contenant des oxydes d'azote, auquel on ajoute éventuellement de l'oxygène ou des mélanges gazeux en contenant, des circuits de recyclage avec extraction partielle des produits principaux et secondaires, et enfin, isole les produits d'oxydation purs.

2. Procédé suivant la revendication 1, caractérisé en ce que l'on introduit ou injecte par soufflage, dans le bouilleur réacteur, sous 40 à 200 mbars, et sous une température appropriée pour l'ébullition du mélange réactionnel,

a) l'acide du procédé avec 40 à 55% en poids de $HNO_3$
b) le composé cyclique à oxyder, les deux produits participants à la réaction étant préchauffés quand la phase de démarrage est écoulée, par les gaz perdus de la réaction,
c) le catalyseur
d) le gaz porteur et éventuellement, en particulier pour l'amorçage de la réaction,
e) de la vapeur d'eau

et qu'en tête de la colonne de rectification, on aspire des oxydes d'azote et de l'oxygène résiduel et les gaz inertes constituant le gaz porteur ainsi que les vapeurs et l'on condense les vapeurs dans des appareils de condensation, dans lesquels on échauffe en même temps les participants introduits à la réaction et envoie ces vapeurs et autres partiellement à nouveau, en retour, en tête de la colonne de rectification, ou les envoie, comme eau contenant des oxydes d'azote, à une installation d'absorption d'acide nitrique, pendant que l'on réchauffe éventuellement à nouveau la partie principale des constituants gazeux à l'aide d'un ventilateur résistant à la corrosion, et en y ajoutant de l'oxygène ou un mélange gazeux le contenant, et la retourne comme gaz porteur dans le fond du bouilleur du réacteur, et qu'on évacue le reste et l'envoie éventuellement à une installation d'absorption d'acide nitrique, et l'on extrait du fond du bouilleur ou du fond du séparateur de buées, continuellement, éventuellement en le chauffant, un liquide d'où l'on sépare, par les procédés de séparation habituels, de l'acide nitrique que l'on ajoute à l'acide du procédé avant de la chauffer dans le condenseur, et qu'enfin, après d'autres opérations de séparation et d'épuration, on obtient, à côté de faibles quantités d'acide nitrique supplémentaire, que l'on peut également utiliser comme complément dans l'acide du procédé, les acides polycarboxyliques sous forme pure.

3. Procédé suivant l'une des revendications 1 et 2, caractérisé en ce que l'on maintient, dans les participants introduits à la réaction, un rapport moléculaire de 1 : 3 à 20, de préférence de 1 : 6 à 12 entre le composé cyclique et $HNO_3$.

4. Procédé suivant l'une quelconque des revendications 1 à 3, caractérisé en ce que l'on utilise, comme acide du procédé, qui doit être introduit dans le réacteur bouilleur, un mélange qui est constitué des

a) acides résiduels provenant de l'épuration des produits d'oxydation que l'on doit isoler et
b) acide dit acide frais,

en amenant la concentration, éventuellement assez faible des acides obtenus suivant a), grâce à une concentration élevée appropriée de l'acide frais b), à la teneur voulue pour l'acide du procédé de 40 à 55% en poids d'$HNO_3$.

5. Procédé suivant l'une quelconque des revendications 1 à 4, caractérisé en ce qu'on utilise, pour

produire la dépression voulue dans le bouilleur, une pompe à anneau à liquide dans le recyclage de liquide en circuit fermé.

6. Procédé suivant l'une quelconque des revendications 1 à 5, caractérisé en ce que l'on fait aspirer de l'air ou de l'oxygène supplémentaire sur le plateau inférieur de la colonne de rectification ou dans le fond du séparateur de buées.

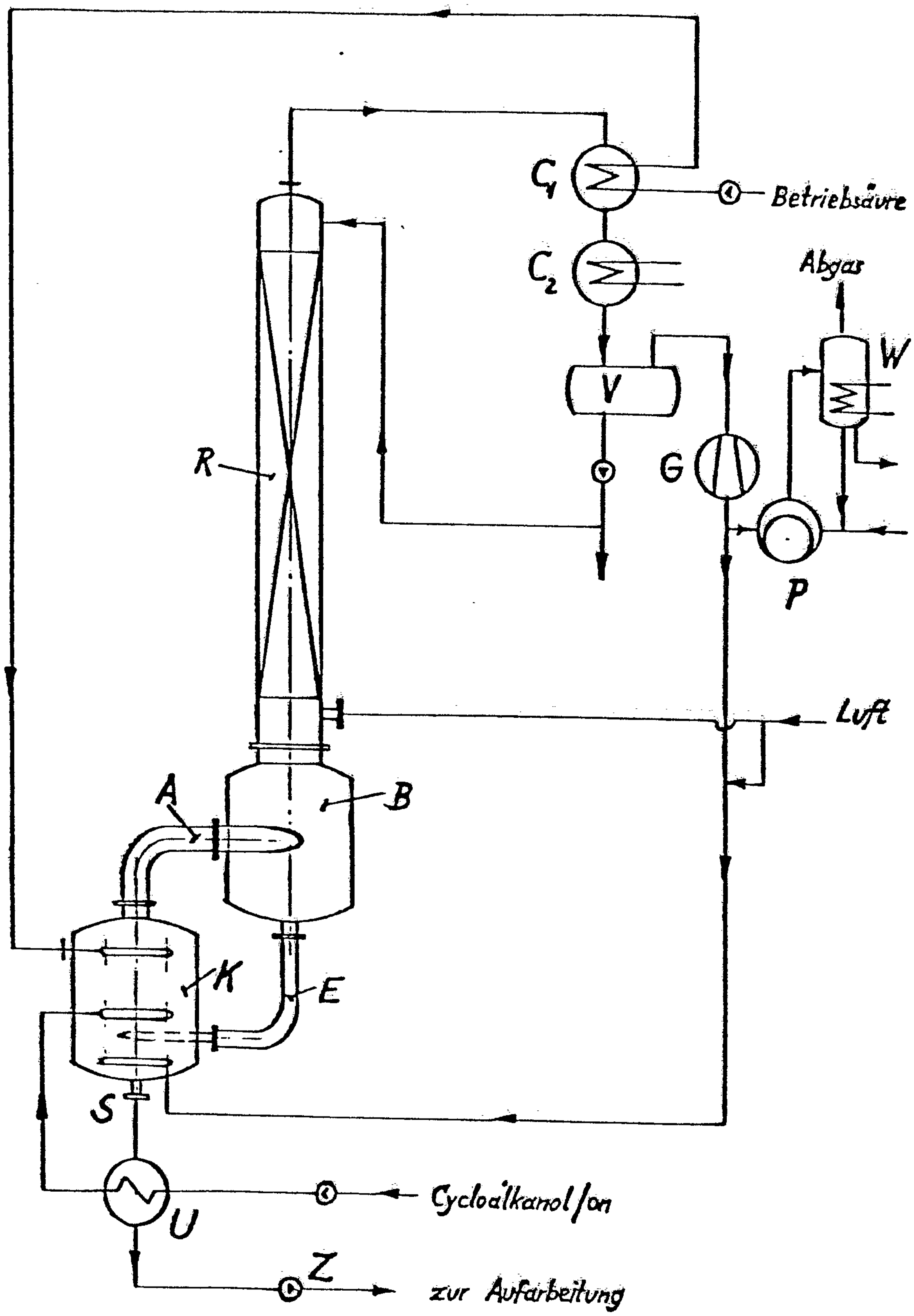
C1
Betriebsäure
C2
Abgas
W
V
R
G
P
Luft
A
B
K
E
S
Cycloalkanol/on
U
Z
zur Aufarbeitung